(19) Europäisches Patentamt
European Patent Office
Office européen des brevets

(11) Publication number: **0 057 489**
**B1**

(12) **EUROPEAN PATENT SPECIFICATION**

(45) Date of publication of patent specification: **12.11.86**

(51) Int. Cl.$^4$: **C 07 H 17/08,** A 61 K 31/70

(21) Application number: **82200130.1**

(22) Date of filing: **01.02.82**

(54) **Thiolic derivatives of erythromycin having therapeutic activity, process for their preparation and pharmaceutical compositions containing them.**

(30) Priority: **02.02.81 FR 8101930**

(43) Date of publication of application:
**11.08.82 Bulletin 82/32**

(45) Publication of the grant of the patent:
**12.11.86 Bulletin 86/46**

(84) Designated Contracting States:
**AT BE CH DE GB LI LU NL SE**

(56) References cited:
**DE-A-2 001 731**
**FR-A-1 465 403**
**FR-A-2 306 704**
**US-A-3 847 896**

**CHEMICAL ABSTRACTS, vol. 93, no. 5, August 1980, page 21, abstract no. 38279f COLUMBUS, Ohio (US) N. KIKUCHI et al.: "Effects of cysteine compounds on antibiotics. 1. Effect of L-cysteine and its derivative on the potency of antiobiotics"**
**CHEMICAL ABSTRACTS CHEMICAL SUBJECT INDEX, vol. 93, Ch-lo December 31, 1980 COLUMBUS, Ohio (US) page 2176CS: L-Cysteine**

(73) Proprietor: **REFARMED, Recherches Pharmaceutiques et Medicales S.A.**
**Dammstrasse 29**
**CH-8702 Zollikon (CH)**

(72) Inventor: **Gonella, Jacques Dr.**
**Bahnhofstrasse 29**
**CH-Zollikon Zurigo (CH)**

(74) Representative: **Dragotti, Gianfranco**
**SAIC BREVETTI s.a.s. Viale Bianca Maria, 15**
**I-20122 Milano (IT)**

**EP 0 057 489 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel thiolic derivatives of erythromycine and of the propionyl ester of erythromycin, mainly salts of erythromycin with acids containing sulfur atoms in their molecule, in form of thioalcohol, thioether or thioester.

The exploitation of the therapeutical properties of thiolic compounds in combination with the properties of antibiotics has been already attempted. However (C.A. *93*, 38279 f) it was found that acetylcysteine and the derivatives thereof have an inhibiting action with respect to the activity of antibiotics.

In turn in Chem. Ab., Chem. Substance Index, vol, 93, CH—10, December 31, 1980 page 2176 Cs, there is discussed in action of N-acetylcysteine on the properties of several chemical substances, amongst which erythromycin is not included.

US—A—384789 relates to the thiolic esters of derivative of erythromycin with bicarboxylic acids, particularly succinic and glutaric acids, having improved taste with respect to erythromycin and a favourable absorption, when orally administered.

It has been now surprisingly found that the compounds of the present invention find therapeutical use in the cases in which erythromycin or the propionyle ester thereof are already used and are generally characterized by a very low toxicity and by a high hematic concentration: these aspects are very important from the therapeutical point of view.

The derivatives according to the present invention have the following general formula:

$$R—X \qquad (I)$$

wherein R is a radical amongst the following radicals:

1) acetylcysteine

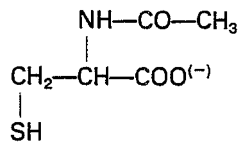

2) carboxymethylcysteine

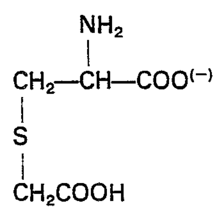

3) thiazolidin-carboxylic acid

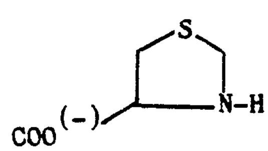

and X is the radical or erythromycin or of the propionyl ester of erythromycin having the formula

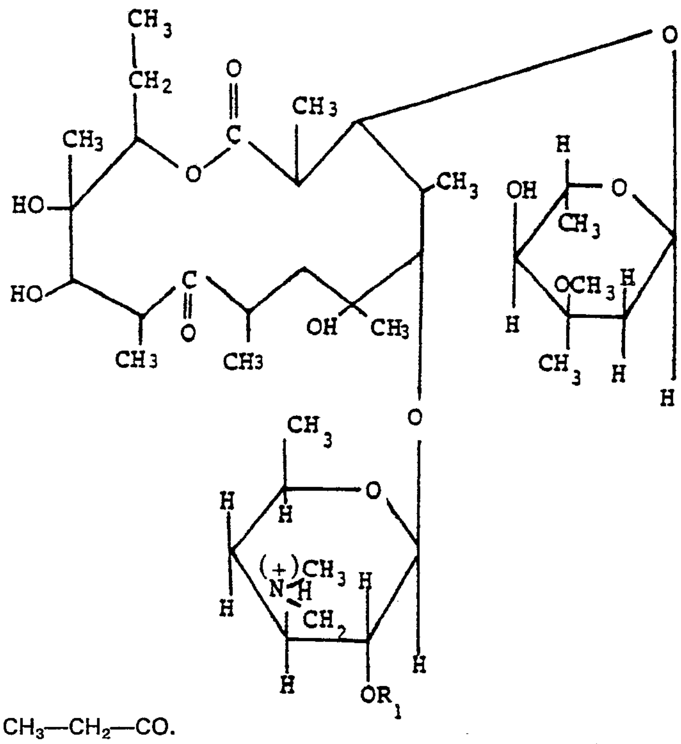

in which $R_1$ is H or $CH_3$—$CH_2$—CO.

The compounds according to the invention are white microcrystalline powders. Generally, the salt containing erythromycin monopropionyl ester is less soluble than the corresponding salt of erythromycin base; for both derivatives, moreover, the solubility increases in solvent mixtures, such as water-ethanol (1—5%), and water-ethylene glycol (1-5%).

Their use is anyhow forseen under all pharmaceutical forms: capsules, solutions injectable preparations, aerosols, creams, powders and suspensions, both for human beings and for verterinary use. Palrticularly for the salt of erythromycin base there are foreseen pharmaceutical preparations of the injectable type or administerable by aerosol, whereas for the derivatives of propionyl erythromycin there are preferred the orally administerable formulations and the suspensions. The compounds are effected by sun light, humidity and heat, and are true salts, both from the chemical and from the physical point of view.

The method for the preparation of the thiolic derivatives of erythromycin or of propionyl ester of erythromycin according to the present invention comprises reacting erythromycin base or the propionyl ester of erythromycin with the acid in a stoichiometrical ratio or in the presence of a slight excess of the antibiotic nucleous and, is characterized in that the reaction is carried out in an organic solvent, at a temperature of between 20 and 40°C and in the presence of water in an amount not greater than 20%.

In fact, it has been found, in a surprising manner, that the presence of water preferably in an amount of 4—5% by volume with respect to the reaction solvent, permits the reaction to be completed with exceedingly good results. On the contrary, water amounts greater than 20% may cause the reaction product to be dissolved again in the aqueous/organic mixture.

The following examples, only given for illustrative purpose, disclose the preparation of the derivatives of the invention.

Example 1

Salt of erythromycin with thiazolidin-4-carboxylic acid

42.32 g of erythromycin base are dissolved in 250 ml of methylisobutylketone until a clear solution is obtained; then, under stirring, the thiazolidin-4-carboxylic acid in finely divided form (7.6 g) is added and the stirring is continued until a total dispersion is obtained over about 30 minutes.

Then 9 mls of water are added dropwise, a vigorous stirring being maintained. A crystalline, white and fine precipitate is slowly formed over 2 hours. After standing for 1 hour the mixture is filtered under vacuum. After drying at the maximum temperature of 40°C, 45 g of solid product are obtained, with a yield of 90.2% of the theoretical value.

In the same manner the corresponding salt is prepared starting from 40.2 g of erythromycin and 9.8 g of carboxymethylcysteine, the product having melting point of 130—140°C and the IR spectrum shown in fig. 1.

Example 2

Salt of propionyl erythromycin with N-acetyl-L-cysteine

20 g of erythromycin propionate are dissolved in 30 mls of acetone and 70 mls of methylisobutylketone.

This solution is added with 4.1 g of N-acetyl-L-cysteine until a complete solution is obtained (under stirring).

The clear, slightly straw-coloured solution, is added, under stirring, with 2.5 mls of water. A precipitate of the salt is slowly formed, within about 2 hours.

After filtration under reduced pressure, 21.5 g of product are obtained with a yield of 80—83% of the theoretical value.

The IR spectrum is shown in fig. 2.

The corresponding salt is likewise prepared starting from 20 g of erythromycin base and 4.45 g of N-acetyl-L-cysteine, and 20 g of the salt are obtained (with a yield of 82% of the theoretical value), the melting point being 128—140°C.

The IR spectrum is shown in fig. 3.

The compounds of the invention have been subjected to the toxicological, pharmacological and pharmacodynamic tests aiming to assess the toxicity (and consequently the possibility of use in the therapeutical field) and the therapeutical properties. In the hereinafter stated results the compounds of the invention are indicated by the following abbreviations:

RV/03 — erythromycin-thiazolidin-carboxylate;
RV/04 — erythromycin-S-carboxymethylcysteinate;
RV/05 — erythromycin-N-acetylcysteinate;
RV/13 — erythromycin-monopropionate-thiazolidin-carboxylate;
RV/14 — erythromycin-monopropionate-S-carboxymethylcysteinate;
RV/15 — erythromycin monopropionate N-acetylcysteinate.

A) Acute toxicity

The acute toxicity was assessed in Swiss white mice by oral and intravenous route. The $LD_{50}$ values have been calculated by the methods of Probits, starting from the mortality recorded 10 days after the treatment.

| Compound | Administration route | $LD_{50}$ mg.kg (reliability limits) |
|---|---|---|
| erythromycin | os | >3000 |
| erythromycin hydrochloride | i.v. | 376.58 (478.78—296.20) |
| RV 03 | os | >3000 |
| RV 03 | i.v. | 491.96 (552.31—438.20) |
| RV 04 | os | >3000 |
| RV 04 | i.v. | 439.46 (545.44—354.08) |
| RV 05 | os | >3000 |
| RV 05 | i.v. | 472.84 (529.08—442.57) |
| RVC 13 | os | 3000 |
| RV 14 | os | 3000 |
| RV 15 | os | 3000 |

B) Antibacterial activity in vitro

The antibacterial activity in vitro of the compounds of the invention was assessed by comparing the different minimum inhibiting concentrations (MIC) with those of erythromycin base and of erythromycin estolate (erythromycin propionate lauryl sulfate) in Gram-positive and Gram-negative strains cultivated on Brain Heart Infusion (Difco). The tested samples of erythromycin estolate, RV 13, RV 14 and RV 15 were previously hydrolyzed according to the prescription given in U.S. Pharmacopoeia, XX edition, page 1347. In the following table 1 the MIC/ml values are indicated corresponding to the lowest concentration capable of inducing a complete inhibition of the bacterial development.

| Strain | | Erythromycin | Erythromycin Estolate | RV 03 | RV 04 | RV 05 | RV 13 | RV 14 | RV 15 |
|---|---|---|---|---|---|---|---|---|---|
| 1) Staph. albus | SS 03 | 0.05 | 0.05 | 0.05 | 0.1 | 0.05 | 0.05 | 0.1 | 0.25 |
| 2) | SS 04 | 0.05 | 0.05 | 0.025 | 0.05 | 0.025 | 0.025 | 0.05 | 0.05 |
| 3) Staph. aureus | SS 07 | 0.025 | 0.205 | 0.025 | 0.025 | 0.025 | 0.025 | 0.025 | 0.025 |
| 4) | SS 08 | 0.025 | 0.025 | 0.025 | 0.05 | 0.025 | 0.05 | 0.05 | 0.025 |
| 5) | SS 09 | 0.1 | 0.05 | 0.05 | 0.025 | 0.1 | 0.1 | 0.1 | 0.05 |
| 6) | SS 10 | 0.1 | 0.2 | 0.1 | 0.2 | 0.025 | 0.1 | 0.2 | 0.05 |
| 7) Strept. haemolyticus | SS 17 | 3 | 3 | 1.5 | 1.5 | 3 | 1.5 | 1.5 | 1.5 |
| 8) | SS 18 | 1.5 | 3 | 1.5 | 1.5 | 0.8 | 1.5 | 0.8 | 3 |
| 9) Strept. faecalis | SS 19 | 0.1 | 0.1 | 0.05 | 0.05 | 0.2 | 0.05 | 0.05 | 0.05 |
| 10) S. lutea ATCC 9341 | | 0.01 | 0.01 | 0.025 | 0.01 | 0.01 | 0.025 | 0.01 | 0.01 |
| 11) B. subtilis ATCC 6633 | | 0.025 | 0.01 | 0.05 | 0.025 | 0.01 | 0.025 | 0.025 | 0.01 |
| 12) | SS 127 | 0.05 | 0.025 | 0.025 | 0.025 | 0.01 | 0.025 | 0.05 | 0.025 |
| 13) B. cereus ATCC 11778 | | 0.4 | 0.8 | 0.08 | 0.2 | 0.4 | 0.8 | 0.4 | 0.4 |
| 14) B anthranis | SS 08 | 0.4 | 0.4 | 0.2 | 0.4 | 0.4 | 0.4 | 0.4 | 0.4 |
| 14bis) | SS 09 | 0.4 | 0.4 | 0.4 | 0.2 | 0.4 | 0.4 | 0.8 | 0.4 |
| 15) Br. melitensis | SS 19 | 3 | 3 | 6 | 6 | 3 | 3 | 3 | 3 |
| 16) Br. abortus | SS 7 | 12 | 6 | 6 | 6 | 12 | 6 | 12 | 6 |
| 17) Citrobacter | SS 05 | 200 | 200 | 200 | 200 | 200 | 200 | 200 | 200 |
| 18) | SS 09 | 200 | 200 | 200 | 200 | 200 | 200 | 200 | 200 |
| 19) S. infantis. | SS 08 | 100 | 100 | 200 | 100 | 100 | 100 | 100 | 100 |

| Strain | | Erythromycin | Erythromycin Estolate | RV 03 | RV 04 | RV 05 | RV 13 | RV 14 | RV 15 |
|---|---|---|---|---|---|---|---|---|---|
| 20) S. hiedelberg | SS 18 | 100 | 100 | 100 | 100 | 100 | 100 | 100 | 100 |
| 21) E. cloacae | SS 07 | 200 | 200 | 200 | 200 | 200 | 200 | 200 | 200 |
| 22) E. aerogenes | SS 011 | 100 | 50 | 50 | 50 | 100 | 50 | 50 | 50 |
| 23) Ps. aeruginosa | SS 10 | 100 | 100 | 100 | 100 | 200 | 100 | 100 | 100 |
| 24 | SS 12 | 200 | 200 | 200 | 200 | 200 | 100 | 100 | 200 |
| 25) E. coli | SS 04 | 100 | 100 | 100 | 100 | 100 | 100 | 100 | 50 |
| 26) | SS 05 | 50 | 100 | 50 | 50 | 25 | 50 | 50 | 100 |
| 27) | SS 06 | 100 | 50 | 50 | 100 | 100 | 50 | 25 | 25 |
| 28) | SS 07 | 50 | 50 | 50 | 25 | 25 | 50 | 50 | 50 |
| 29) Pr. mirabilis | SS 09 | 200 | 200 | 200 | 200 | 200 | 200 | 200 | 200 |
| 30) Pr. vulgaris | SS 10 | 200 | 200 | 200 | 200 | 200 | 200 | 200 | 200 |
| 31) Kl. aerogenes | SS 71 | 50 | 100 | 50 | 50 | 50 | 100 | 100 | 100 |
| 32) Kl. pneumoniae | SS 04 | 100 | 100 | 100 | 100 | 50 | 100 | 100 | 100 |
| 33) Sr. marcescens | SS 03 | 200 | 200 | 100 | 200 | 200 | 100 | 200 | 200 |
| 34) C. diphtheriae | SS 19 | 0.01 | 0.025 | 0.025 | 0.205 | 0.025 | 0.01 | 0.01 | 0.02 |
| 35) D. pneumoniae | SS 23 | 0.05 | 0.05 | 0.025 | 0.05 | 0.05 | 0.025 | 0.05 | 0.05 |
| 36) N. gonorreae | SS 27 | 0.8 | 0.8 | 0.8 | 0.8 | 0.8 | 0.8 | 0.8 | 0.8 |
| 37) N. meningitidis | SS 04 | 0.8 | 1.5 | 1.5 | 1.5 | 0.8 | 0.8 | 0.8 | 0.8 |
| 38) H. influentiae | SS 02 | 1.5 | 1.5 | 1.5 | 1.5 | 0.8 | 1.5 | 1.5 | 1.5 |
| 39) H. pertussis | SS 03 | 0.4 | 0.4 | 0.4 | 0.8 | 0.2 | 0.2 | 0.4 | 0.2 |

| Strain | | Erythromycin | Erythromycin Estolate | RV 03 | RV 04 | RV 05 | RV 13 | RV 14 | RV 15 |
|---|---|---|---|---|---|---|---|---|---|
| 40) H. tubercolosis | SS 03 | 50 | 50 | 25 | 50 | 50 | 50 | 50 | 50 |
| 41) C. albicans | SS 04 | 100 | 200 | 200 | 100 | 100 | 100 | 200 | 200 |
| 42) Cl. tetani | SS 17 | 0.4 | 0.2 | 0.4 | 0.2 | 0.2 | 0.2 | 0.2 | 0.2 |

C) Antibacterial activity in vivo

Experimental infection induced by Staph. aureus and D. pneumoniae

Swiss white mice of 20 g body weight were used, infected by parenteral administration of lethal amounts of pathogenic genes of Staph. aureus SS 07 and D. pneumoniae SS 23. The protecting activity expressed as $ED_{50}$ mg/kg was assessed for erythromycin base, erythromycin estolate, RV 03, RV 04, RV 05, RV 13, RV 14, and RV 15 administered by oral route as well as for erythromycin ethyl succinate, RV 03 RV 04 and RV 05 administered by the subcutaneous route.

The antibiotics were administered at the time of injecting the infecting germ and 6 and 24 hours later. On the basis of the survival after 7 days, the $ED_{50}$ values were determined, as reported in the following table II and III, wherein the reliability limits are indicated in brackets.

TABLE II

Experimental infection by Staph. aureus SS 07 ($ED_{50}$ mg/kg of erythromycin)

| Compound | Administration route | $ED_{50}$ mg/kg (reliability limits) |
|---|---|---|
| erythromycin ethyl succinate | s.c. | 1.03 (1.34—0.79) |
| RV 03 | s.c. | 1.00 (1.36—0.73) |
| RV 04 | s.c. | 1.00 (1.24—0.80) |
| RV 05 | s.c. | 1.33 (1.61—1.10) |
| erythromycin | os | 2.09 (2.36—1.86) |
| erythromycin estolate | os | 2.20 (2.44—1.99) |
| RV 03 | os | 2.14 (2.42—1.89) |
| RV 04 | os | 2.31 (2.55—2.09) |
| RV 05 | Os | 2.08 (2.38—1.83) |
| RV 06 | Os | 2.02 (2.32—1.75) |
| RV 13 | os | 2.20 (2.44—1.99) |
| RV 14 | os | 2.26 (2.49—2.05) |
| RV 15 | os | 2.10 (2.49—1.92) |

TABLE III

Experimental infection by D. pneumoniae SS 23 ($ED_{50}$ mg/kg of erythromycin)

| Compound | Administration route | $ED_{50}$ mg/kg (reliability limits) |
|---|---|---|
| erythromycin ethyl succinate | s.c. | 54.23 (69.76—42.16) |
| RV 03 | s.c. | 47.98 (59.13—38.93) |
| RV 04 | s.c. | 60.17 (70.74—51.18) |
| RV 05 | s.c. | 50.22 (62.33—40.46) |
| erythromycin | os | 113.53 (147.33—87.48) |
| erythromycin estolate | os | 127.89 (156.42—104.57) |
| RV 03 | os | 119.21 (142.60—99.65) |
| RV 04 | os | 119.21 (152.88—92.95) |
| RV 05 | os | 119.31 (150.05—94.87) |
| RV 13 | os | 113.88 (144.73—89.60) |
| RV 14 | os | 123.74 (148.77—102.92) |
| RV 15 | os | 110.90 (135.28—90.92) |

D) Pharmacodynamics
a) In the rat.

The absorption by oral route of the different salt of erythromycin was investigated in Sprague-Dawley male rats which were administered, in groups of 6 animals fasted since 12 hours, with erythromycin, erythromycin estolate, RV 03, RV 04, RV 05, RV 13, RV 14, RV 15 at the dose of 100 mg/kg (expressed as erythromycin) by oral route (gastric probing).

The blood samples were taken 0.30, 1, 2, 3, 4, 5 and 6 hours after the administration. For the dosing the microbiological method, and as the test micro-organism, B. subtilis were used. The experimental results are reported in the following table IV:

TABLE IV
Hematic levels in the rat (average plus standard error)
mcg/ml after .... .... hours

| Compound | No. of animals | 0.30 | 1 | 2 | 3 | 4 | 5 | 6 |
|---|---|---|---|---|---|---|---|---|
| erythromycin | 6 | 0.71 | 1.03 | 1.15 | 1.05 | 0.37 | 0.26 | 0.03 |
| | | 0.19 | 0.23 | 0.24 | 0.22 | 0.10 | 0.08 | — |
| erythromycin estolate | 6 | 0.68 | 1.74 | 2.49 | 1.49 | 0.61 | 0.37 | 0.04 |
| | | 0.07 | 0.14 | 0.35 | 0.22 | 0.10 | 0.05 | 0.01 |
| RV 03 | 6 | 0.58 | 0.71 | 1.30 | 0.77 | 0.59 | 0.36 | 0.07 |
| | | 0.14 | 0.14 | 0.34 | 0.17 | 0.14 | 0.10 | 0.02 |
| RV 04 | 6 | 0.50 | 0.71 | 1.30 | 1.04 | 0.68 | 0.43 | 0.15 |
| | | 0.14 | 0.17 | 0.29 | 0.28 | 0.21 | 0.15 | 0.08 |
| RV 05 | 6 | 0.44 | 0.83 | 1.41 | 1.03 | 0.59 | 0.40 | 0.08 |
| | | 0.07 | 0.22 | 0.38 | 0.28 | 0.16 | 0.12 | 0.01 |
| RV 13 | 6 | 0.81 | 1.80 | 2.47 | 1.10 | 0.72 | 0.52 | 1.12 |
| | | 0.07 | 1.10 | 0.23 | 0.17 | 0.11 | 0.11 | 0.05 |
| RV 14 | 6 | 0.72 | 1.85 | 2.55 | 1.39 | 0.65 | 0.54 | 0.07 |
| | | 0.10 | 0.09 | 0.12 | 0.08 | 0.10 | 0.10 | 0.01 |
| RV 15 | 6 | 0.85 | 1.90 | 2.68 | 1.19 | 0.55 | 0.44 | 0.03 |
| | | 0.23 | 0.08 | 0.15 | 0.08 | 0.03 | 0.04 | — |

B) In the healthy volunteer

Group of 5 healthy volunteers, fasted since 24 hours, were administered by oral route with erythromycin estolate, RV 13, RV 14, RV 15, at a dose of 500 mg of erythromycin.

The blood samples were taken 0.30, 1, 2, 3 and 4 hours after the administration.

For the dosing, the microbiological method using a B. subtilis strain was adopted.

The results are indicated in table V:

TABLE V
Hematic levels in the healthy volunteer (average plus standard error)
mcg/ml after .... .... hours

| Compound | No. of animals | 0.30 | 1 | 2 | 3 | 4 | 5 | 6 |
|---|---|---|---|---|---|---|---|---|
| erythromycin estolate | 5 | 0.27 | 0.58 | 2.10 | 1.41 | 1.31 | — | — |
| | | 0.05 | 0.09 | 0.17 | 0.07 | 0.08 | — | — |
| RV 13 | 5 | 1.16 | 1.67 | 2.21 | 1.67 | 1.29 | — | — |
| | | 0.03 | 0.07 | 0.11 | 0.09 | 0.10 | — | — |
| RV 14 | 5 | 1.20 | 1.78 | 2.28 | 1.60 | 1.21 | — | — |
| | | 0.07 | 0.04 | 0.06 | 0.14 | 0.06 | — | — |
| RV 15 | 5 | 1.10 | 1.58 | 2.17 | 1.72 | 1.38 | | |
| | | 0.09 | 0.05 | 0.14 | 0.09 | 0.06 | | |

E) Mucolytic activity

There was used in the method described by Quevauviler et al (Thérapie 22,485,1967) according to which a bronchial hypersection is induced after exposure of the animal to an aerosol treatment with $SO_2$. The test was carried out on Spraque Dawley male rats.

All rats were daily subjected to $SO_2$ inhalations, at a 0.03% concentration. After 50 hours of inhalation, the animals were divided in groups comprising 10 animals each. One group was not treated (control animals), whereas the other animals were treated, two hours per day, for 15 day consecutively with inhalations of $SO_2$ and with erythromycin estolate and RV 13, RV 14, RV 15, by oral route at the dose of 500 mg/kg as well as with erythromycin ethyl succinate and RV 03, RV 04, RV 05, by intramuscular route at the dose of 250 mg/kg. The day after the last treatment, the anesthetized animals were sacrificed, and the lungs were removed and prepared for the microscopical and macroscopical examination. The results are indicated in the following Table VI.

TABLE VI

Qualitative and quantitative evaluation of the obstruction of the bronchial tract (% incidence)

| Treatment | Administration route | Total obstruction compact plug | nodular mass | pimply mass | Partial obstruction |
|---|---|---|---|---|---|
| $SO_2$ (control) | — | 40 | 13.33 | 6.66 | 26.66 |
| $SO_2$ + erythromycin estolate 500 mg/kg | oral | 46.66 | 20 | 6.66 | 20 |
| $SO_2$ + RV 13 500 mg/kg | oral | 20 | 6.66 | 13.33 | 20 |
| $SO_2$ + RV 14 500 mg/kg | oral | 26.66 | 6.66 | 6.66 | 13.33 |
| $SO_2$ + RV 15 50 mg/kg | oral | 20 | 13.33 | 6.66 | 6.66 |
| $SO_2$ + erythromycin ethyl succinate 250 mg/kg | i.m. | 53.33 | 26.66 | 13.33 | 20 |
| $SO_2$ + RV 03 250 mg/kg | i.m. | 26.66 | 20 | 6.66 | 6.66 |
| $SO_2$ + RV 04 250 mg/kg | i.m. | 20 | 13.33 | 6.66 | 13.33 |
| $SO_2$ + RV 05 250 mg/kg | i.m. | 20 | 13.33 | 6.66 | 6.66 |

Thereafter the histological examination of both control and treated animals was carried out.

1) Control animals subjected to a $SO_2$ inhalation

The macroscopical bronchial obstructions as detected by the hystological examination correspond to a mucus mass admixed with fibrin and infiltrated with polynuclear elements. The hypersection does equally concern the peripheral bronchioles and the aveoles. As regards the bronchial epithelium there is revealed a proliferation of cup-shaped cells. The hyperplasy of the main bronchus appears as a stratification of 7 to 8 layers, associated to a bronchial hypertrophy.

2) Animals treated with erythromycin estolatel at the dose of 500 mg/kg by oral route and with erythromycin ethyl succinate at the dose of 250 mg/kg by intramuscular route

From the histological examination it appears that the bronchial obstruction is formed by abundant mucus admixed with fibrin. Due to the irritating effect of $SO_2$, the bronchial epithelium reacts through a proliferation of cells, mainly cup-shaped cells. The hyperplasy is associated in a number of cases to a bronchial hypertrophy. In some cases, mucus secreting cells appear in the peripheral bronchioles.

3) Animals treated with RV 13, RV 14, RV 15, at the dose of 500 mg/kg by oral route.

In the animals having, under macroscopical examination, a free non-obstructed bronchial tract, the histological examination revealed the presence of bronchi having almost normal appearance. An epithelial hyperplasy with a number of cup-shaped cells and reduced intro-bronchial muco-purulents masses was detected. The hystological appearance of the obstructed bronchial tracts is fully like that of the control animals.

4) Animals treated with RV 03, RV 04, RV 05, at the dose of 250 mg/kg by intramuscular route.

The histological examination as carried out in the animals which under macroscopical examination had free non obstructed bronchi, revealed a practically normal appearance. In some animals, the presence of an epithelial hyperplasy was detected with cup-shaped cells associated to intra-bronchial muco-purulent masses. The histological examination of the animals showing an obstructed bronchial tract, revealed an appearance like that of the control animals.

Conclusions

From the tess it results that the erythromycin salts are devoid of toxicity when administered by oral route. When injected, they can be slightly toxic and have however $LD_{50}$ values comparable with that of erythromycin.

From the test of antibacterial activity in vitro it results that the erythromycin salts are active in a proportion like that of the erythromycin base.

From the test of antibacterial activity in vivo and form the pharmacodynamic test it results that the compounds RV 03, RV 04, RV 05 are poorly absorbed by oral route and in a rate like that of erythromycin base.

The propionyl derivatives RV 13, RV 14, RV 15 are absorbed by oral route in a rate practically analogous to that of erythromycin estolate. Contrarywise to erythromycin estolate, the drugs according to the invention are endowed with a good mucolytic activity which is revealed in the animals treated by $SO_2$ inhalation.

**Claims**

1) Thiolic derivatives of erythromycin and of propionyl ester of erythromycin having the formula:

$$R—X \qquad (1)$$

where in R represents a radical selected in the class comprising

1) acetylcysteine

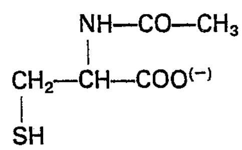

2) carboxymethylcysteine

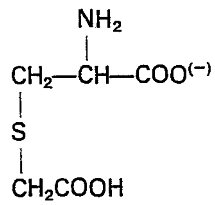

3) thiazolidin-carboxylic acid

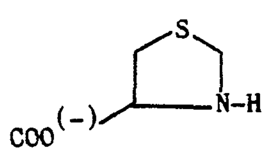

and X is the radical or erythromycin or of the propionyl ester of erythromycin having the formula:

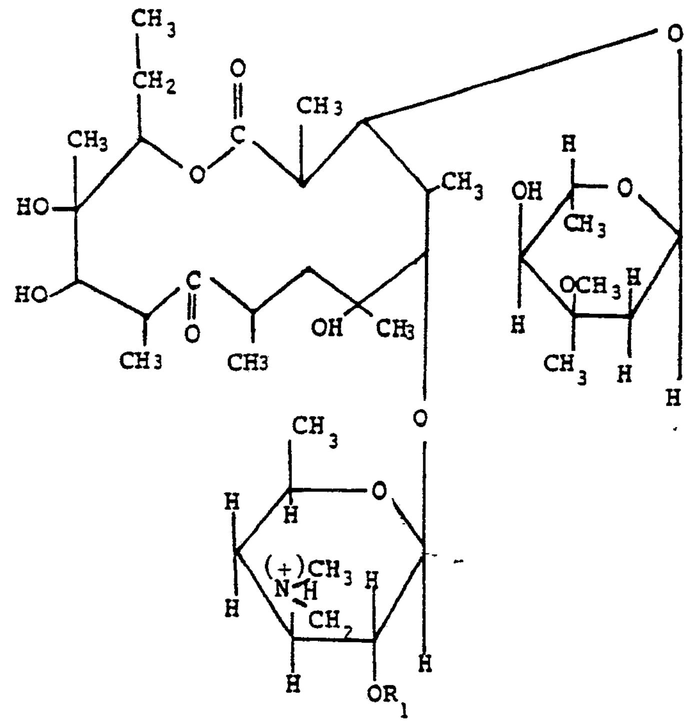

in which $R_1$ is H or $CH_3$—$CH_2$—CO.

2. Erythromycin salt with acetylcysteine according to claim 1.

3. Erythromycin salt with thiazolidin-4-carboxylic acid according to claim 1.

4. Erythromycin salt with carboxymethylcysteine according to claim 1.

5. Salt of propionic ester of erythromycin with acetylcysteine according to claim 1.

6. Salt of propionyl ester of erythromycin with thiazolidin-4-carboxylic acid according to claim 1.

7. Salt of propionyl ester of erythromycin with carboxymethylcysteine according to claim 1.

8. A process for the preparation of the thiolic derivatives of erythromycin and of the propionyl ester of erythromycin, wherein erythromycin base or the propinyl ester thereof is reacted with the desired acid, characterized is that the reaction is carried out in organic solvent, at a temperature of 20 to 40°C and in the presence of water.

9. A process according to claim 8, characterized in that the water present in the reaction solvent is no more than 20% by volume of the reaction solvent.

10. A process according to claim 9, characterized in that said water volume is 4 to 5% of that of the reaction solvent.

11. A process according to claim 10, characterized in that said organic solvent is acetone or methylisobutylketone.

12. Pharmaceutical preparation, characterized by containing, as the active ingredient a thiolic derivative according to claim 1, as well as vehicles and fillers of usual type and acceptable from the pharmaceutical point of view.

13. Pharmaceutical preparation, characterized by containing, as the active ingredient a derivative according to claim 1.

14. Pharmaceutical preparation according to claims 12 and 13 characterized by being in a form permitting the oral administration particularly in form of capsules and suspensions, and by containing as the active ingredient an erythromycin derivative according to claim 1, as well as the usual excipients and vehicles.

15. Pharmaceutical preparation according to claims 12 and 13, characterized by being in form permitting the administration by parental route and by aerosol, and by containing as the active ingredient a derivative of the propionyl ester of erythromycin according to claim 1, as well as the usual excipients and vehicles.

**Patentansprüche**

1. Schwefel enthaltende Erythromycin und Erythromycinpropionylester-Derivate der Formel:

R—X

worin R ein Radikal bedeutet, das aud der Klass, ausgewählt ist, welche:

1) Acetylcystein

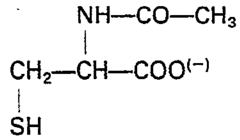

2) Carboxymethylcystein

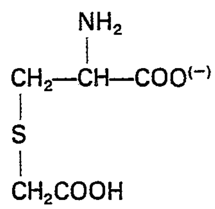

3) Thiazolidin-4-carboxylsäure

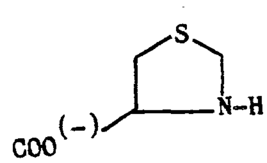

umfast, und X das Erythromycin oder Erythromycinpropionylester Radikal der Former:

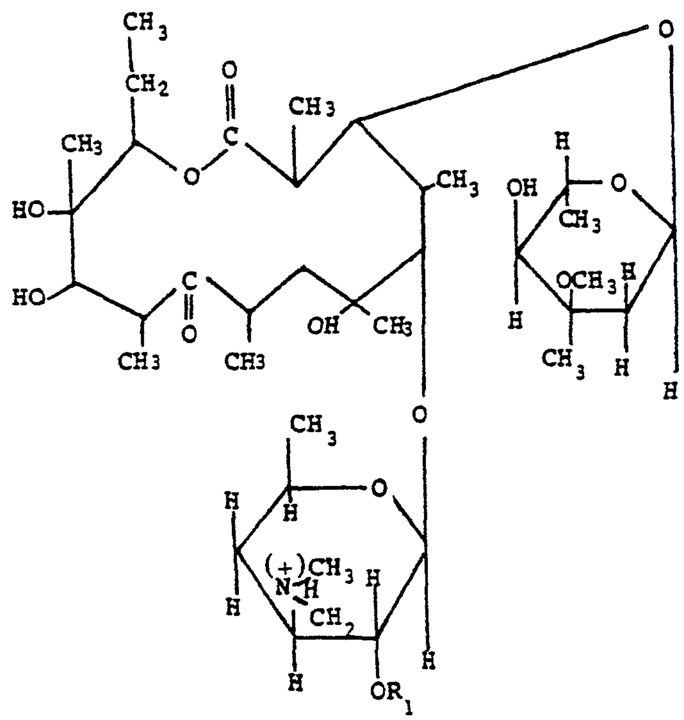

ist, worin $R_1$ H oder $CH_3—CH_2—CO$ ist.

2. Salz des Erythromycins mit Acetylcystein nach Anspruch 1.
3. Salz des Erythromycins mit Thiazolidin-4-Carboxylsäure nach Anspruch 1.
4. Salz des Erythromycins mit Carboxylmethylcystein nach Anspruch 1.
5. Salz des Erythromycinpropionylesters mit Acetylcystein nach Anspruch 1.
6. Salz des Erythromycinpropionylesters mit Thiazolidin-4-Carboxylsäure nach Anspruch 1.
7. Salz des Erythromycinpropionylesters mit Carboxymethylcystein nach Anspruch 1.
8. Verfahren zur Herstellung von Schwefel enthaltenden Erythromycin und

Erythromycinpropionylester-Derivaten, wobei Erythromycinbase oder der Propionylester davon mit der gewünschten Säure umgesetzt wird, dadurch gekennzeichnet, dass die Umsetzung bei einer Temperatur von 20 bis 40°C und in Gegenwart von Wasser in einem organischen Lösungsmittel durchgeführt wird.

9. Verfahren nach Anspruch 8, dadurch gekennzeichnet, dass das im Umsetzungslösungmittle enthaltende Wasser nich mehr als 20 Vol.% des Umsetzungslösungsmittels ausmacht.

10. Verfahren nach Anspruch 9, dadurch gekennzeichnet, dass das besagte Wasservolumen 4 bis 5% desjenigen des Umsetzungslösungsmittels beträgt.

11. Verfahren nach Anspruch 10, dadurch gekennzeichnet, dass das gesagte organische Lösungsmittel Azeton oder Methylisobutylketon ist.

12. Pharmazeutische Zubereitung, dadurch gekennzeichnet, dass sie als wirksamen Bestandteil ein Schwefel enthaltendes Derivat nach Anspruch 1, sowie übliche und pharmazeutisch annehmbare Träger und Füllstoffe enthält.

13. Pharmazeutische Zubereitung, dadurch gekennzeichnet, dass sie als wirksamen Bestandteil ein Derivat nach Anspruch 1 enthält.

14. Pharmazeutische Zubereitung nach den Ansprüchen 12 und 13, dadurch gekennzeichnet, dass die sich in einer, die orale Verabreichung gestattenden Form, insbesondere in Form von Kapseln oder Suspensionen befindet und dass sie als wirksamen Bestandteil ein Erythromycin-Derivat nach Anspruch 1, sowie die üblichen Bindemittel und Träger enthält.

15. Pharmazeutische Zubereitung nach den Ansprüchen 12 und 13, dadurch gekennzeichnet, dass sie sich in einer, die parenterale bzw. die Aerosol-Verabreichung gestattenden Form befindet und dass sie als wirksamen Bestandteil ein Erythromycinpropionylester-Derivat nach Anspruch 1, sowie die ülichen Bindemittel und Träger enthält.

**Revendications**

1. Dérivés thioliques de l'érythromycine et du propionylester propionique de l'érythromycine ayant pour formule:

$$R—X \qquad (I)$$

où R représente un radical choisi dans la classe comprenant:

1) acétylcystéine

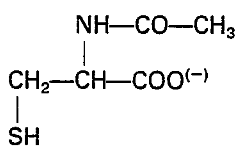

2) carbosyméthylcystéine

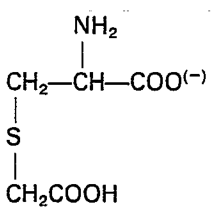

3) acide-thiazolidine-4-carboxylique

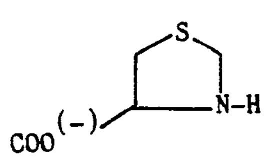

et X représente le radical de l'érythromycine ou du propionylester de l'érythromycine, ayant pour formule:

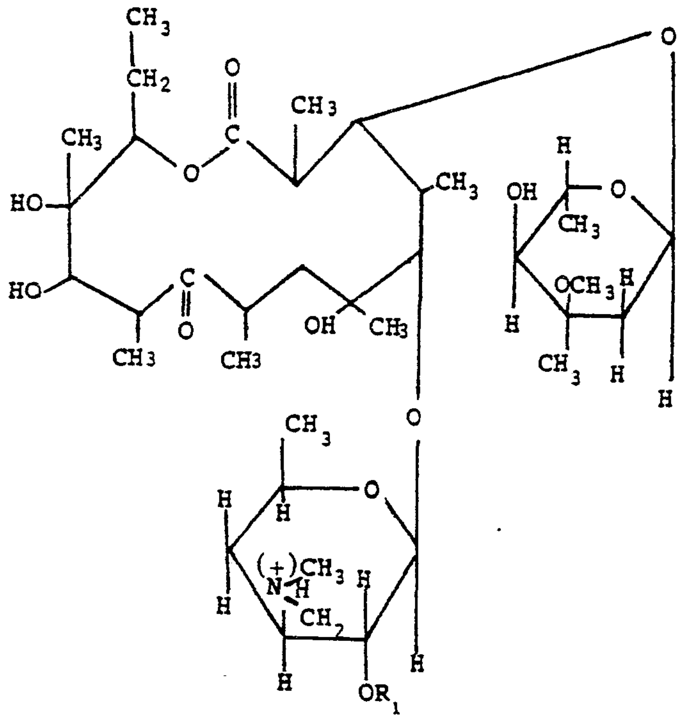

où $R_1$ représente H, ou $CH_3—CH_2—CO$.

2. Sel d'érythromycine avec acétylcystéine suivant la revendication 1.

3. Sel d'érythromycine avec acide thiazolidine-4-carboxylique suivant la revendication 1.

4. Sel d'érythromycine avec carboxyméthylcystéine suivant la revendication 1.

5. Sel d'ester propionque d'érythromycine avec acétylcysteine suivant la revendication 1.

6. Sel due propionyl d'érythromycine avec acide thiazolidine-4-carboxylique suivant la revendication 1.

7. Sel du propionylester d'érythromycine avec carboxyméthylcystéine suivant la revendication 1.

8. Procédé de préparation des dérivés thioliques d'érythromycine et du propionylester d'érythromycine dans lequel l'érythromycine base ou son propionylester réagit avec l'acide souhaité, caractérisé en ce que la réaction s'effectue dans un solvant organique, à une température comprise entre 20 et 40°C et en présence d'eau.

9. Procédé de préparation suivant la revendication 8, caractérisé en ce que l'eau présente dans le solvant de réaction d'est pas supérieure à 20% en volume du solvant de réaction.

10. Procédé de préparation suivant la revendication 9, caractérisé en ce que ledit volume d'eau est de 4 à 5% de celui du solvant de réaction.

11. Procédé de préparation suivant la revendication 10, caractérisé en ce que ledit solvant organique est de l'acétone ou du méthylisobutylcétone.

12. Préparation pharmaceutique, caractérisée en ce qu'elle contient pour ingrédient actif, un dérivé thiolique suivant la revendication 1, ainsi que des véhicules et des supports de type usuel, et acceptables d'un point de vue pharmaceutique.

13. Préparation pharmaceutique caractérisée en ce qu'elle contient pour ingrédient actif un dérivé suivant la revendication 1.

14. Préparation pharmaceutique suivant l'une des revendications 12 et 13, caractérisée en ce qu'elle se présente sous une forme qui lui permet dêtre administrée par voie orale en particulier sous forme de capsules et de suspensions, et en ce qu'elle contient pour ingrédient actif un dérivé de l'érythromycine suivant la revendication 1, ainsi que les excipients et véhicules usuels.

15. Préparation pharmaceutique suivant l'une des revendications 12 et 13, caractérisée en ce qu'elle se présente sous un forme qui lui permet d 'être administrée par voie parentale et par aérosols et en ce qu'elle contient pour ingrédient actif, un dérivé du propionylester d'érythromycine selon la revendication 1 ainsi que les excipients et véhicules usuels.

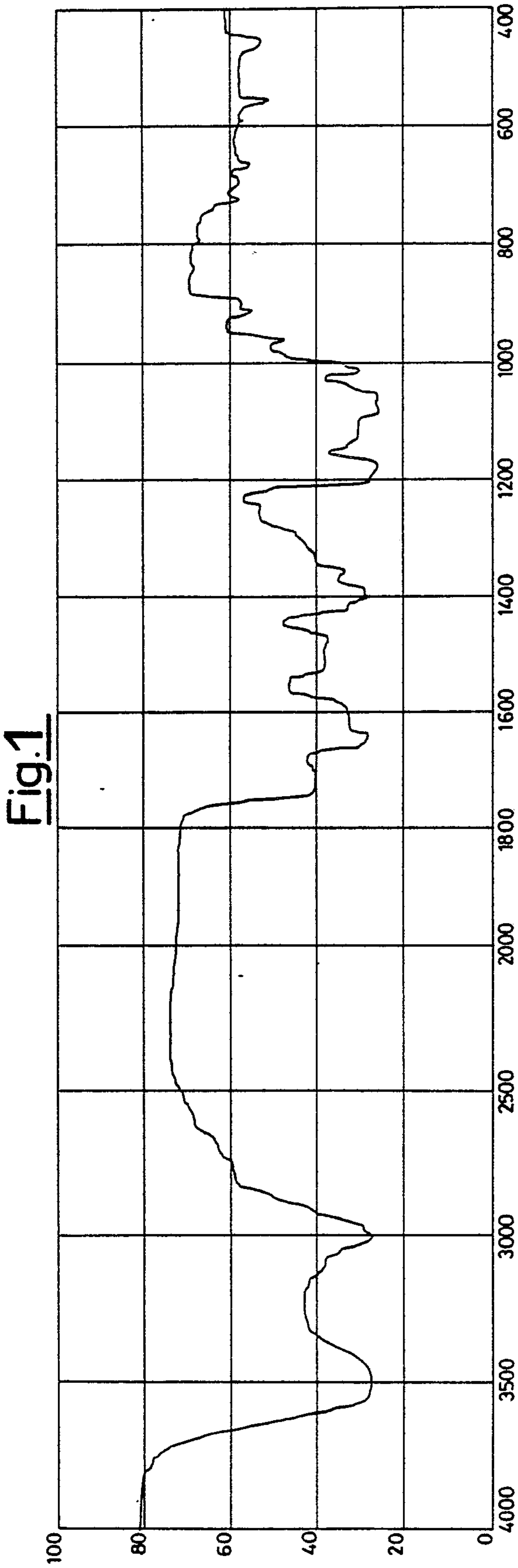

400
600
800
1000
1200
1400
1600
1800
2000
2500
3000
3500
4000
100
80
60
40
20
0


Fig.1

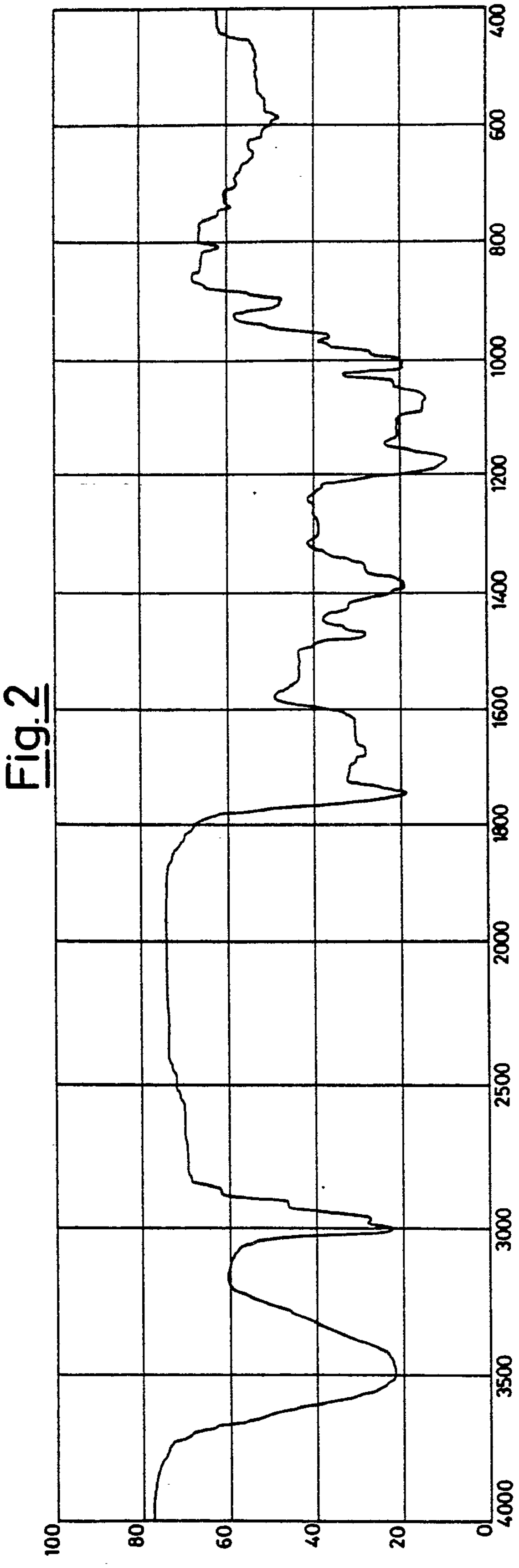
100
80
60
40
20
0
4000
3500
3000
2500
2000
1800
1600
1400
1200
1000
800
600
400

Fig. 2

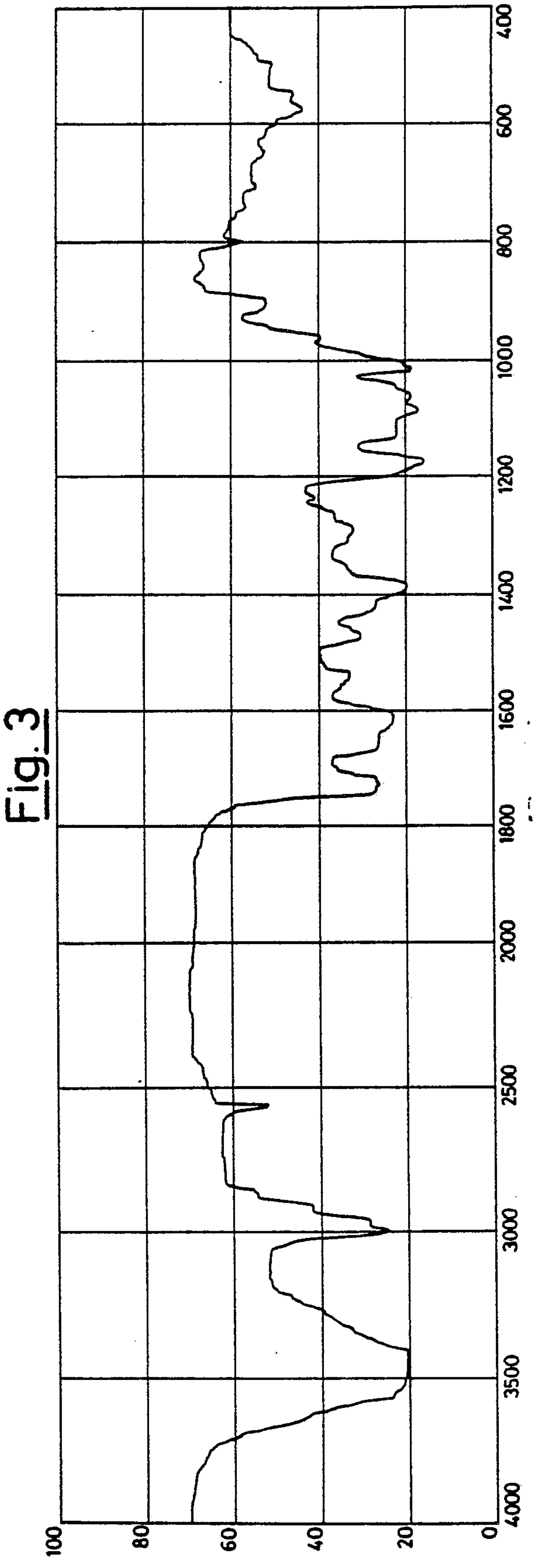
100
80
60
40
20
0
4000
3500
3000
2500
2000
1800
1600
1400
1200
1000
800
600
400

Fig. 3